# EUROPEAN PATENT SPECIFICATION

(11) **EP 0 411 132 B1**
(45) Date of publication and mention of the grant of the patent: **10.05.1995**
(21) Application number: 89911103.3
(22) Date of filing: 05.10.1989
(51) Int. Cl.: A61N 5/06

(54) **HEATING EQUIPMENT USING LASER BEAM**
HEIZUNGSVORRICHTUNG UNTER VERWENDUNG EINES LASERSTRAHLS
EQUIPEMENT CHAUFFANT UTILISANT UN FAISCEAU LASER

(30) Priority: 05.10.1988 JP 252462/88
(43) Date of publication of application: 06.02.1991
(73) Proprietor: S.L.T. JAPAN CO, LTD., Tokyo 102 (JP)
(72) Inventor: DAIKUZONO, Norio, Chiba-ken 290-02 (JP)
(74) Representative: Bloch, Gérard
(86) International application number: JP8901024
(87) International publication number: WO9003826

(56) References cited:
- EP-A- 0 152 766
- WO-A-84/02069
- GB-A- 2 154 761
- JP-A- 0 006 113
- JP-A- 1 204 662
- JP-A- 5 995 065
- JP-A- 6 040 069
- JP-A-63 216 579
- JP-A-63 260 577
- JP-A-63 318 933
- US-A- 4 643 186
- US-A- 4 654 024
- US-A- 4 662 368

## Description

### Technical Field

This invention relates to a laser-light irradiation apparatus for use in thermal therapy and, more particularly, to a laser-light irradiation apparatus with a liquid seal member containing laser-light scattering particles, by which laser-light emitted from optical fiber is scattered during being passed through the liquid seal member, prior to being irradiated on living tissues for heating the tissues.

### Prior Art

Applications of laser-light beams are conspicuous in the medical field these days. Lately, (localized) thermal therapy is drawing special attention as a carcinostatic therapy. According to this method, cancer tissues are destroyed by keeping same at a temperature of about 42-44°C for 10-25 minutes by irradiation with laser-light. The effectiveness of this method has been reported by the inventors in the bulletin of Japan Society of Laser Medicine, vol. 6, No. 3 (January 1986), pp. 71-76 & 347-350.

On the other hand, considerable attention has been paid to laser-chemical therapies including the method reported in 1987 by Dougherty et al of the United States. According to this method, 48 hours after an intravenous injection of a hematoporphyrin derivative (HpD), weak laser-light such as argon laser or argon pigment laser is irradiated against a target area of treatment. Whereupon oxygen of the primary term which has a strong carcinostatic action is produced by HpD. Since then, there have been published various reports in this regard, including the one in the bulletin of Japan Society of Laser Medicine, vol. 6, No. 3, pp 113-116. In this connection, it has also been known in the art to use "pheophobide a" as a photo-reactant. Further, YAG laser has been put into use as a laser-light source.

Any way, for heating living tissues, it has been the conventional practice to irradiate the target tissues with laser-light emitted from a probe pierced into the target tissues, controlling the intensity of the laser-light emission in such a manner as to maintain the tissues in a temperature range of 42-44°C.

However, the above-described localized heating treatment, which employs a pair of a laser-light probe and a temperature sensor, has a temperature distribution as shown in Fig. 14, in which the temperature with a peak at the center of the probe Pr drops sharply toward marginal portions of the irradiated area. If the center portion of the irradiated area is maintained in the above-mentioned temperature range, the irradiation is effective only for the tissues in the central portion but not for the tissues in other areas remote from the center portion. This necessitates to effect the irradiation at a number of different points in each treatment. Accordingly, there has been a problem that the treatment can be made only to a particular local area during a limited time of operation.

Under these circumstances, the inventor proposed in his Japanese Patent Application Laid-Open No. 63-216579 a laser-light irradiation apparatus which has a plural number of probes positioned abreast of each other. Although not sufficient, this device has been found to be effective for uniformalizing the temperature distribution and broadening the area of treatment. However, difficulties are encountered in inserting a plural number of optical fiber systems being arranged in parallel, which are connected to the probes respectively, into narrow spaces such as esophagus, blood vessels and the like, coupled with an increase in cost which is inevitable due to the necessity for providing branch conduction passages which transmit laser-light from a main laser-light conduction passage to the respective fiber systems, and a group of switches required for the respective branch conduction passages.

Known from the prior-art document US-A-4612938 is a laser-light irradiation apparatus as recited in the preamble of claim 1.

It is therefore a main object of the present invention to provide an inexpensive laser-light irradiation apparatus which permits thermal therapy of a broad area in a single treatment with substantially uniform temperature distribution among various points of the irradiated area, as well as treatments in narrow spaces of various tubular organs.

### Disclosure of the Invention

In the present invention, there is provided a laser-light irradiation apparatus according to claim 1.

According to the present invention, prior to irradiating living tissues, the laser-light from the emitting member is repeatedly scattered by the laser-light scattering particles and absorbed by the laser-light absorbing particles as it is transmitted through the laser-light scattering liquid in the liquid seal member. As a result of the scattering, the vital tissues are irradiated with the laser-light coming from all directions and from the entire effective irradiation area of the liquid seal member. This permits to irradiate a broader area and, as a result of the absorbing action, to distribute the irradiated energy uniformly to heat up evenly the target area of the thermal treatment.

Further, since a broad area can be irradiated by the laser-light irradiation apparatus with the single emitting member, it becomes possible to effect the heating treatment in narrow spaces in various organs, blood vessels or other hollow tubular organs to a sufficient degree, in addition to a significant cost reduction as compared with the above-mentioned device employing a plural number of laser-light transmission systems.

Moreover, the present invention is extremely effective for heating a target area in local heating therapy or in laser-chemical therapy. Needless to say, it is also useful for ordinary coagulation purposes.

In a case where the liquid seal member is formed of a flexible material, it is useful for the treatment of blood vessels constricted by deposition of cholesterol, spreading a constricted portion and softening cholesterol by expanding the liquid seal member. Further, since the liquid seal member is flexible, even if the surface of the tissues is not flat but roughened, the liquid member can be fit perfectly to the entire surface of the tissues.

On the other hand, in the preferable embodiment of the present invention, a laser-light reflecting surface is provided on the inner surface of the liquid seal member. By so doing, since the laser-light is reflected at the reflecting surface in the liquid seal member, the laser-light emitted from a surface, which has no reflecting surface, can be concentratedly irradiated on the treated tissue.

In another preferable embodiment of the present invention, a temperature sensor like a thermocouple is provided on the surface of the liquid seal member. A laser-light power to transmit can be controlled by detecting the energy of the laser-light irradiated on the treated tissue with the temperature sensor.

In another preferable embodiment of the present invention, when the laser-light irradiation apparatus is inserted into hollow tubular tissues, in order to prevent the inner surface of the tissues from being injured, a guide wire of the apparatus, which serves as a guide member, is inserted into the tissues before inserting the other parts of the apparatus.

### Brief Description of the Drawings

Fig. 1 is a longitudinal section of an embodiment of the present invention;
Fig. 2 is a view taken on line II -II of Fig. 1;
Fig. 3 is a schematic illustration showing a laser-light irradiation apparatus in use for heating a constricted portion of blood vessel;
Fig. 4 is a schematic illustration of a laser-light irradiation apparatus which is provided with a reflecting surface on a balloon;
Figs. 5 and 6 are fragmentary longitudinal sections of a laser-light irradiation apparatus which is provided with a non-flexible liquid seal member;
Fig. 7 is a longitudinal section of a laser-light irradiation apparatus employing a guide wire;
Fig. 8 is a longitudinal section of an embodiment in which a probe itself serves as a laser-light emitting member;
Figs. 9 and 10 are schematic sectional views of an embodiment which is arranged to scatter the laser-light at the surface of the probe;
Fig. 11 is a longitudinal section of another embodiment;
Fig. 12 is a longitudinal section of still another embodiment;
Fig. 13 is a schematic sectional view of an embodiment which is arranged to scatter the laser-light at the scattering surface layer of the probe; and
Fig. 14 is a temperature distribution diagram of a conventional device.

### The Best Mode to Carry out the Invention

Now, the present invention is described more particularly by way of the embodiments shown in the drawings.

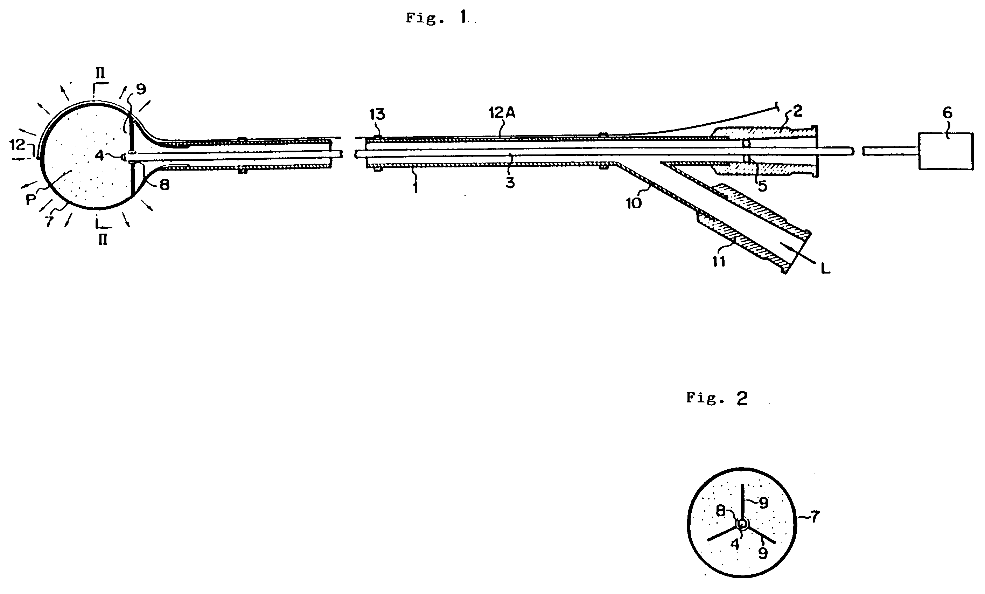

Referring to Fig. 1, there is illustrated a first embodiment of the present invention, which is provided with a flexible guide tube 1 of a synthetic resin material or the like and of a predetermined length. A stopper cock 2 is attached to the base end of the guide tube 1. Inserted in the guide tube 1 is optical fiber 4 which is protected by a coaxial protective tube 3 and has an O-ring 5 fitted between the protective tube 3 and the stopper cock 2 for sealing purposes. The base end of the optical fiber is optically connected to a laser-light generator 6.

On the other hand, fixed to the fore end of the guide tube 1 is a balloon 7 which serves as a liquid seal member of the present invention and which is made of a flexible and laser-light transmissible material such as rubber latex, silicon rubber, polyethylene, polypropyrene or the like. In Fig. 1, the balloon 7 is shown in an inflated state. Elastic members, for example, three elastic members consisting of rubber strips 9 are provided in equidistant positions between the inner surface of the balloon 7 and a sleeve member 8 which is fixedly fitted on the fore end portion of the protective tube 3.

Communicably branched from the base end portion of the guide tube 1 is a liquid feed tube 10 which has a stopper cock 11 attached to the base end thereof. A temperature sensor, for example, a thermocouple or thermocouples 12 are fixed in a suitable position or positions in the fore end portion of the balloon 7, a lead wire 12A of the thermocouple 12 being passed around the surface of the balloon 7 and extended toward the base end of the guide tube 1. The lead wire 12A is fixed on the outer surface of the guide tube 1 at predetermined intervals by the use of suitable fixation means, for example, by fastening bands 13, and connected to a temperature control unit which is not shown.

With the balloon 7 in deflated state, the laser-light irradiation apparatus of the above-described construction is inserted into the living body toward a target area of the treatment. In this instance, the guide tube 1, the protective tube 3 and the optical fiber 4 are flexible, so that the apparatus can be inserted smoothly even along a bent or curved portion which might exist in the path of insertion. Next, a scattering liquid L is fed to the apparatus through the stopper cock 11 which is located outside the living body.

The laser-light scattering liquid may be water or an organic liquid such as glycol, which contains a multitude of laser-light scattering particles P therein. The scattering particles are preferred to be in the form of powder rather than granules and to have a particle size not larger than 100»m, more preferably, not larger than 15»m. The particles are of a material which has laser-light scattering effect chosen among diamond, sapphire, glass, ceramics, plastics, and metals.

Upon feeding the laser-light scattering liquid L, the balloon 7 is inflated, stretching the rubber strips 9 in such a manner as to hold the protective tube 3 and optical fiber 4 in a center position within the guide tube 1 and the balloon 7, while preventing the balloon 7 from being transformed by balancing action relative to the pressure of the liquid L or part of the balloon 7 from approaching the fore end of the optical fiber 4 and being ruptured by the laser-light from the optical fiber 4.

With the balloon 7 in inflated state, the laser-light from the laser generator 6 is transmitted to the optical fiber 4 and emitted from the fore end of the optical fiber 4. The emitted laser-light is repeatedly scattered by the scattering particles P as it is passed through the balloon 7, and irradiated on the living tissues from all directions as shown in Fig. 1 to heat the tissues.

The heating temperature is detected by the thermocouple 12, and the output signal of the thermocouple is supplied to the temperature control unit which controls the laser-light power to be transmitted to the optical fiber 4 from the laser generator 6 to control the heating temperature to a desired level.

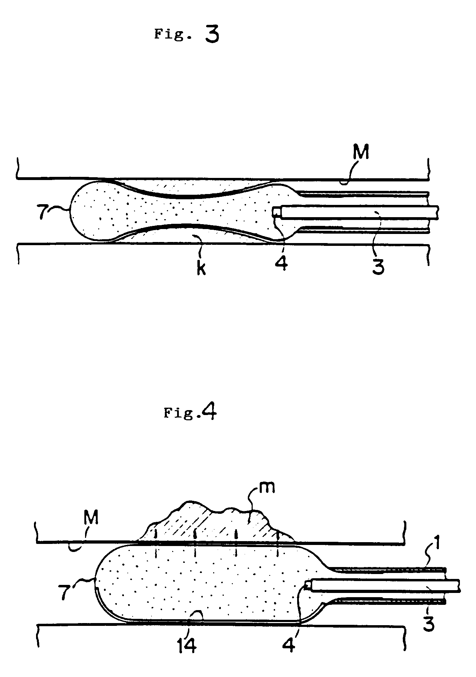

On the other hand, for example, in case of the treatment of a blood vessel M which is constricted by cholesterol k as illustrated in Fig. 3, the balloon 7 is positioned in the constricted portion to eliminate the constriction, softening cholesterol k by heating while spreading or destroying cholesterol k by the pressure of the inflated balloon 7.

In a case where it is desired to heat only cancer tissues m which exist in a deviated position on one side of a blood vessel M as shown in Fig. 4, without heating normal tissues, the laser-light can be concentratedly irradiated only on the cancer tissues m by providing a reflecting surface 14 on the inner surface of the balloon 7. The reflecting surface can be formed by coating or vapor deposition of a material which is not transmissible of laser-light, for example of paint, aluminum, gold or the like.

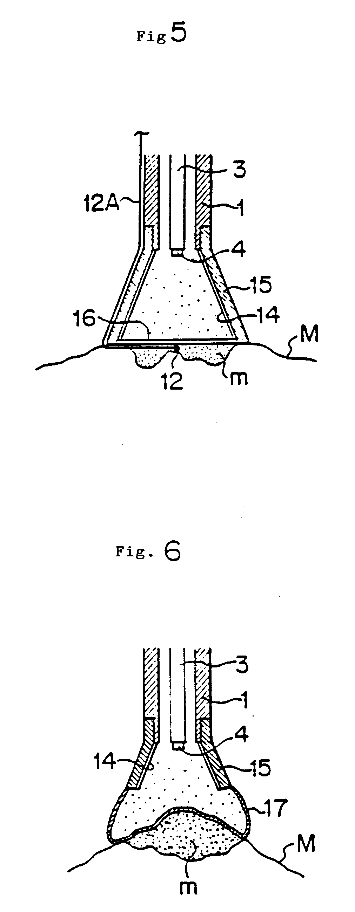

In the present invention, normally the liquid seal member is desired to be flexible as a whole. However, as illustrated in Fig. 5, a non-flexible metal skirt member 15, preferably with a reflecting surface 14 on the inner side thereof, may be attached to the fore end of the guide tube 1. A laser-light transmissible member 16 of glass, quartz, or other material is fixed to the bottom of the skirt member 15. The liquid seal member is constituted by the combination of the skirt member 15 and the laser-light transmissible member 16. Cancer tissues m are irradiated by the laser-light, which is scattered to be reflected on the reflecting surface 14 in the liquid seal member and which is passed through the liquid seal member so as to be emitted from only the transmissible member 16.

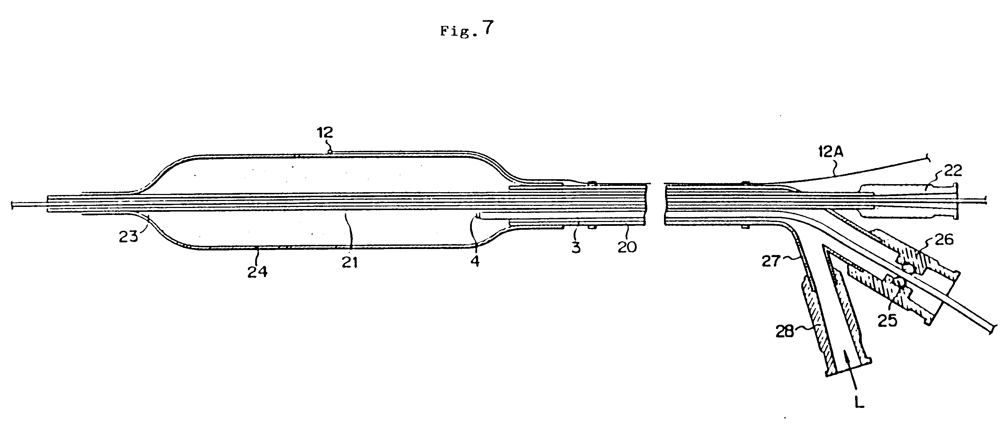

Alternatively, as illustrated in Fig. 6, a flexible shoe member 17 similar to the above-described balloon 7 may be provided at the fore end of the skirt member 15 to irradiate the laser-light through the shoe member 17 which is deformed in conformity with the surface configuration of cancer tissues m. In the embodiments shown in Figs. 5 and 6, it is also preferred to provide a temperature sensor like the thermocouple 12.

Referring now to Fig. 7, there is shown an embodiment which is suitable for use in treatments within the tissues which are bent to a large extent or easily injured like a blood vessel.

In this embodiment, an insert tube 21 of a synthetic resin or other flexible material is inserted in an elongated flexible guide tube 20, and a flexible guide wire 23 is passed through the insert tube 21 and a stopper cock 22 attached to the base end of the insert tube 21. This guide wire 23 consists of an extremely fine metal wire or filament which is more flexible in its fore end portion, and may use a commercial product of such a nature.

Indicated at 24 is a sleeve-like flexible tube which is formed of a material similar to the above-described balloon 7 and which has its both ends securely fixed to the fore ends of the insert tube 21 and the guide tube 20. Optical fiber 4 which is sheathed in a protective tube 3 is inserted in the guide tube 20, with the fore end of the optical fiber 4 extended into the tube 24 while its base end is drawn out of the guide tube 20 through a stopper cock 26 with an O-ring 25 and optically connected to a laser generator in like manner as described in the embodiment of Fig. 1. Further, communicably branched from the base end of the guide tube 20 is a liquid feed tube 27 with a stopper cock 28 through which the afore-mentioned scattering liquid L is fed to the liquid seal member under pressure.

In this embodiment, after inserting the guide wire 23 into the insert tube 21 outside a living body, the guide wire 23 alone is inserted first into a blood vessel or other organ to be treated. Next, inserting portions of the apparatus other than the guide wire 23 are pushed in. By so doing, the inserting portions are guided along the pre-inserted guide wire 23, bent along a curved portion of the blood vessel if any. Accordingly, there is no possibility of the inserting portions rupturing the blood vessel when pushed thereinto.

Then, after confirming that the tube 24 has been inserted to a target portion for the treatment by suitable means, the scattering liquid L is fed under pressure to inflate the tube 24 into intimate contact with the inner surfaces of the blood vessel, and laser-light is emitted from the fore end of the optical fiber 4 to heat the blood vessel.

In this instance, a reflecting surface may also be formed on the inner surface of the tube 24 which constitutes the liquid seal member in this embodiment.

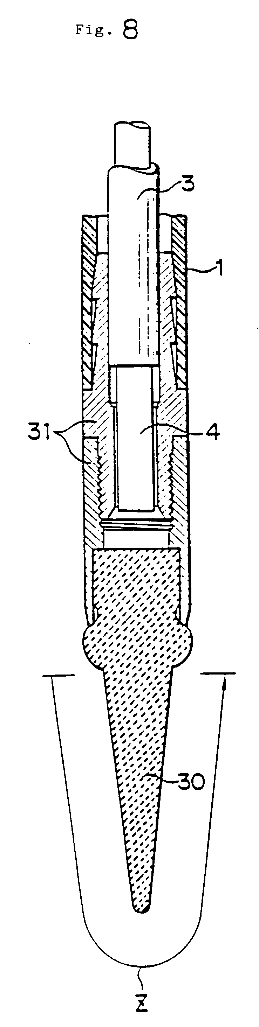

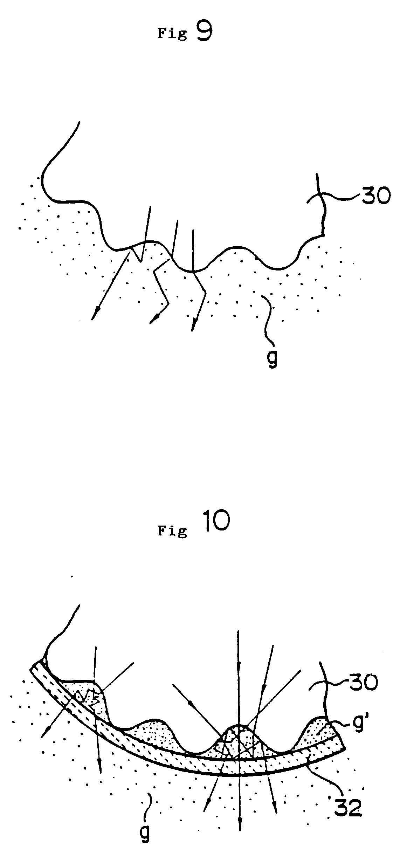

On the other hand, while the optical fiber 4 itself is used as a laser-light emitting member in the foregoing embodiments, a laser-light emitting probe 30 of a laser transmissible material such as quartz, sapphire or the like may be attached in front of the optical fiber 4, for example, by the use of a coupling 31 as shown particularly in Fig. 8, the laser-light from the optical fiber 4 being led to the probe 30 and emitted into the laser-light scattering liquid L through the probe 30. In this instance, as shown in Fig. 9, a roughened surface may be formed in part or on the whole of the zone Z denoted in Fig. 8 to emit the laser-light in a multitude of directions. Part of the emitted laser-light is reflected back toward the roughened surface by the laser-light scattering particles g'. As a result of this shuttle phenomenon, the laser-light is uniformly emitted from the whole roughened surface.

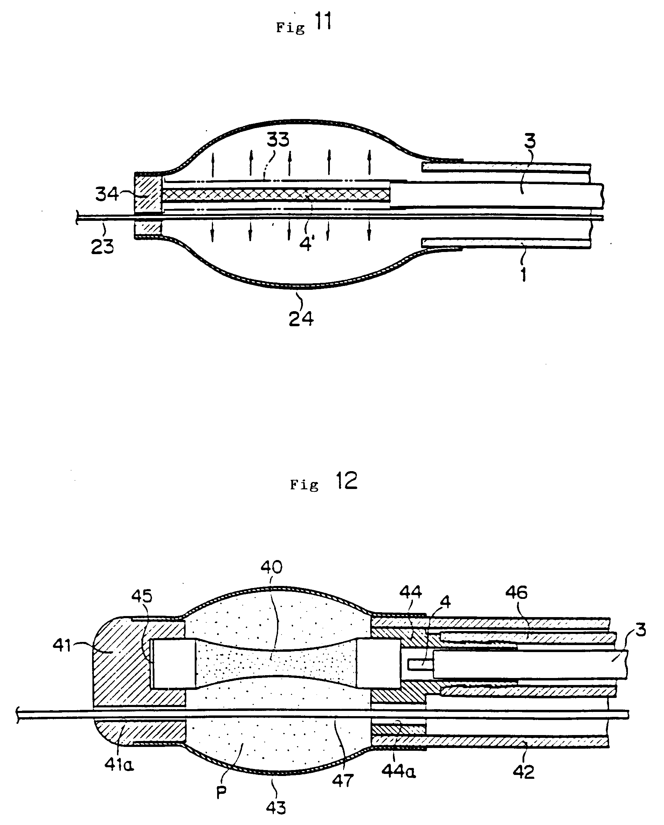

Further, as shown in Fig. 10 of another modification, after coating the dispersion of laser-light scattering particles g on the roughened surface, a laser-light transmissible film 32, which partially or entirely reflects laser-light, might be formed on the coating of the particles g for uniform laser-light emission.

Referring to Fig. 11, there is shown the modification of the embodiment of Fig. 7. In this modification, the optical fiber 4' is exposed over an increased length and provided with a roughened surface in like manner as shown in Fig. 9 or the combination of the roughened surface and the laser-light transmissible film 32 in like manner as shown in Fig. 10 to emit the laser-light from its entire circumferential surface.

The optical fiber 4 or probe 30 is not necessarily required to be in direct contact with the laser-light scattering liquid L, and may be provided with a cover tube 33 of quartz or other laser-light transmissible material as indicated by imaginary line in Fig. 11.

In Fig. 11, the reference numeral 34 denotes a fitting member of a metallic or other laser-light non-transmissible material, which securely holds the fore end of the tube 24 while permitting passage therethrough of the guide wire 23. If there is possibility of the irradiation of the laser-light rupturing the guide wire 23, the reflecting layer of the laser-light such as a gold-plating layer might be formed on the guide wire 23.

In the embodiment of Fig. 11, the core of the optical fiber is exposed over an increased length to emit the laser-light from the entire circumferential surface.

A probe 40 having a form, for example, of a sandglass may be provided at the fore end of the optical fiber 4 to emit the laser-light from the entire surface of the probe 40, as shown in Fig. 12.

In the apparatus of Fig. 12, a fitting member 41, which is rounded at its fore circumferential surface, is provided at the fore end of the apparatus. A guide tube 42 of a synthetic resin material or the like is provided at the base end of the apparatus. A laser-light transmissible balloon 43 is provided between the fitting member 41 and the guide tube 42, while the both ends of the balloon 43 are fixed on them. A metallic coupling 44 is held at the fore end of the guide tube 42, and the probe 40 is provided fixedly between the coupling 44 and the fitting member 41. An aluminum- or gold- plating layer 45 is formed at the fore end surface of the probe 40 to reflect the laser-light totally at the plating layer 45. Further, the optical fiber 4 is held by the coaxial coupling 44 behind the probe 40. The reference numeral 46 is a protective tube.

On the other hand, a flexible guide wire 47 is passed through the guide tube 42, a penetrating hole 44a of the coupling 44, the balloon 43 and a penetrating hole 41a of the fitting member 41. A multitude of laser-light scattering particles P are contained and sealed in the balloon 43 in like manner as described in the aforementioned embodiments.

In this apparatus having the above construction, the laser-light emitted from the fore end of the optical fiber 4 comes into the probe 40 at its back end, and then during passing through the inside of the probe 40, the laser-light is emitted from the surface of the probe 40. The laser-light from the surface of the probe 40 repeats scattering by the laser-light scattering particles P and is irradiated on the living tissues through the balloon 43.

In this embodiment, a laser-light scattering layer 40a may be provided, for example, on the hollow surface alone of the probe 40 in order to elevate the degree of the laser-light scattering.

The laser-light scattering layer 40a can be formed as follows; The probe 40 is dipped in a solution of a volatile liquid, for example, alcohol in which the laser-light scattering particles such as sapphire, silica, alumina and so on are dispersed. These laser-light scattering particles have a larger refractive index than that of the material of the probe 40. After this procedure, the probe 40 is pulled up from the solution and at least the surface of the probe 40 is heated at a temperature near to the melting point of the laser-light scattering particles. Then, the surface alone of the laser-light scattering particles are partially fused, the particles adhere to each other at their fused surfaces, ane they adhere to the surface of the probe 40.

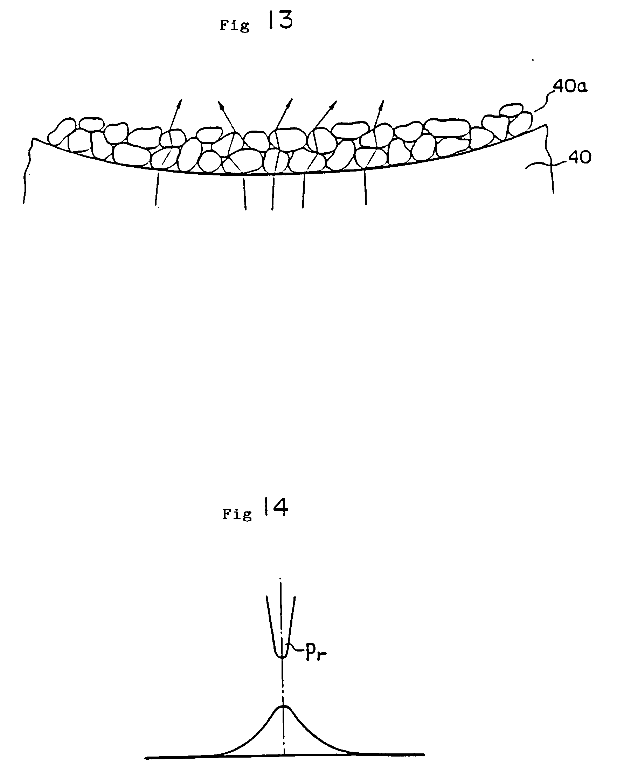

As the result of forming the surface layer 40a, the degree of scattering action is elevated, because the laser-light is scattered at the scatering surface of 40a, as shown in Fig. 13.

In the present invention particles chosen among carbon, graphite, iron oxides, and manganese oxide which can absorb laser-light, are contained in the laser-light scattering liquid in the balloon together with the laser-light scattering particles, so that the laser-light is converted to thermal energy and the heating effect is elevated.

Further, in the present invention, the degree of laser-light scattering action by the laser-light scattering liquid can be varied by selecting a suitable kind or content of the laser-light scattering particles. The scattering liquid may be in the form of a suspension or colloidal liquid if desired.

### Industrial Utilization

It will be appreciated from the foregoing description that, the laser-light irradiation apparatus related to the present invention is extremely effective for heating in localized thermal therapy, laser-chemical therapy and the like. Further, the apparatus can be applied for ordinary coagulation purposes.

## Claims

1. A laser-light irradiation apparatus, comprising :
a laser-light emitting member (4) ;
a liquid seal member (7,24) formed of a laser-light transmissible material and arranged to circumvent said laser-light emitting member (4) ;
a laser-light scattering liquid (L) held in said liquid seal member (7,24) and containing a multitude of laser-light scattering particles therein, characterized by the fact that
said laser-light scattering liquid (L) contains a multitude of laser-light absorbing particles chosen among carbon, graphite, iron oxide, and manganese oxide, together with said laser-light scattering particles, chosen among diamond, sapphire, glass, ceramics, plastics, and metal.

2. A laser-light irradiation apparatus as defined in claim 1, wherein said liquid seal member (7) is formed of a flexible material and inflatable upon receipt of a supply of said laser-light scattering liquid (L).

3. A laser-light irradiation apparatus as defined in claim 1, wherein said liquid seal member (7) has a reflecting layer (14) formed on the inner surface thereof.

4. A laser-light irradiation apparatus as defined in claim 1, wherein said liquid seal member (7) has a temperature sensor (12) fixed on the surface thereof.

5. A laser-light irradiation apparatus as defined in claim 1, which has an irradiating portion (4) constructed to be inserted along a pre-inserted guide wire (23).

6. A laser-light irradiation apparatus as defined in claim 1, wherein said laser-light emitting member (4) is optical fiber.

7. A laser-light irradiation apparatus as defined in claim 1, wherein said laser-light emitting member is a probe (30), which is provided at the fore end of optical fiber (4) and which can transmit laser-light.

8. A laser-light irradiation apparatus as defined in claim 7, wherein said probe (30) has a laser-light scattering layer formed on the surface thereof.

## Patentansprüche

1. Laserlichtbestrahlungsvorrichtung, mit :
einem Laserlichtemissinonsteil (4);
einem Flüssigkeitsabschirmteil (7, 42) aus laserlichtdurchlässigem Material, das das Laserlichtemissionsteil (4) umgibt;
einer Laserlichtstreuflüssigkeit (L) im Flüssigkeitsabschirmteil (7, 24) mit vielen Laserlichtstreupartikeln, dadurch gekennzeichnet, daß
die Laserlichtstreuflüssigkeit (L) viele laserlichtabsorbierende Partikel, ausgewählt aus Kohlenstoff, Graphit, Eisenoxid, und Manganoxid, gemeinsam mit Laserlichtstreupartikeln, ausgewählt aus Diamant, Saphir, Glas, Keramik, Kunststoff, und Metall aufweist.

2. Laserlichtbestrahlungsvorrichtung nach Anspruch 1, wobei das Flüssigkeitsabschirmteil (7) aus einem flexiblen Material und durch Aufnahme von Laserlichtstreuflüssigkeit (L) aufblasbar ist.

3. Laserlichtbestrahlungsvorrichtung nach Anspruch 1, wobei das Flüssigkeitsabschirmteil (7) einem auf seiner Innenoberfläche ausgebildete Reflektionsschicht (14) besitzt.

4. Laserlichtbestrahlungsvorrichtung nach Anspruch 1, wobei das Flüssigkeitsabschirmteil (7) einen aus seiner Oberfläche angebrachten Temperatursensor (12) besitzt.

5. Laserlichtbestrahlungsvorrichtung nach Anspruch 1, mit einem Bestrahlungsabschnitt (4), der zum Einbringen entlang eines voreingebrachten Leitdrahtes (32) ausgebildet ist.

6. Laserlichtbestrahlungsvorrichtung nach Anspruch 1, wobei das laserlichtemittierende Teil (4) eine Lichtleitfaser ist.

7. Laserlichtbestrahlungsvorrichtung nach Anspruch 1, wobei das laserlichtemittierende Teil eine Sonde (30) am Vorderende der Lichtleitfaser (4) ist und Laserlicht leiten kann.

8. Laserlichtbestrahlungsvorrichtung nach Anspruch 7, wobei auf der Oberfläche der Sonde (30) eine Laserlichtstreuschicht ausgebildet ist.

## Revendications

1. Appareil de rayonnement de lumière laser, comprenant :
un élément émetteur de lumière laser (4);
un joint hydraulique (7, 24) constitué d'une matière transmettant la lumière laser et agencé de façon à entourer l'élément émetteur de lumière laser (4);
un liquide (L) diffusant la lumière laser contenu dans ledit joint hydraulique (7, 24) et contenant une multitude de particules diffusant la lumière laser,
caractérisé par le fait que ledit liquide diffusant la lumière laser (L) contient une multitude de particules absorbant la lumière laser choisies parmi le carbone, le graphite, l'oxyde de fer et l'oxyde de manganèse conjointement avec lesdits particules diffusant la lumière, qui sont choisies parmi le diamant, le saphir, le verre, les céramiques, les plastiques et les métaux.

2. Appareil de rayonnement de lumière laser selon la revendication 1, dans lequel le joint hydraulique (7) est constitué d'une matière souple et peut gonfler lorsqu'il reçoit dudit liquide diffusant la lumière laser (L).

3. Appareil de rayonnement de lumière laser selon la revendication 1, dans lequel une couche réfléchissante (14) est formée sur la face intérieure du joint hydraulique (7).

4. Appareil de rayonnement de lumière laser selon la revendication 1, dans lequel un capteur de température (12) est fixé à la surface du joint hydraulique (7).

5. Appareil de rayonnement de lumière laser selon la revendication 1, qui a une portion rayonnante (4) conçue de façon à être introduite le long d'un fil guide pré-introduit (23).

6. Appareil de rayonnement de lumière laser selon la revendication 1, dans lequel l'élément émetteur de lumière laser (4) est une fibre optique.

7. Appareil de rayonnement de lumière laser selon la revendication 1, dans lequel l'élément émetteur de lumière laser est une sonde (30) qui est prévue à l'extrémité avant d'une fibre optique (4) et peut transmettre la lumière laser.

8. Appareil de rayonnement de lumière laser selon la revendication 7, dans lequel une couche diffusant la lumière laser est formée à la surface de la sonde (30).
